# EUROPÄISCHE PATENTSCHRIFT

(11) **EP 1 036 556 B1**
(45) Veröffentlichungstag und Bekanntmachung des Hinweises auf die Patenterteilung: **29.09.2004**
(21) Anmeldenummer: 98122268.0
(22) Anmeldetag: 24.11.1998
(51) Int. Cl.: A61K 7/06

(54) **Verwendung von Ceramiden zur Haarpflege**
Use of ceramides for hair care
Utilisation de céramides pour le soin des cheveux

(43) Veröffentlichungstag der Anmeldung: 20.09.2000
(73) Patentinhaber: KPSS-Kao Professional Salon Services GmbH, 64297 Darmstadt (DE)
(72) Erfinder: Grit, Mustafa, Dr., 64579 Gernsheim (DE); Theis, Heinz, Dr., 64354 Reinheim (DE); Dubowoj, Polina, 64319 Pfungstadt (DE); Uellner, Martin, 64295 Darmstadt (DE)

(56) Entgegenhaltungen:
- EP-A- 0 720 847
- EP-A- 0 845 256
- WO-A-97/14401
- DATABASE WPI Week 199832 Derwent Publications Ltd., London, GB; AN 1998-365522 XP002171122 & JP 10 001423 A (KANEBO LTD.), 6. Januar 1998 (1998-01-06)
- DE PAEPE ET AL.: "Ceramides/Cholesterol/Free fatty acids containing cosmetics..." SÖFW JOURNAL, Bd. 122, Nr. 4, 1996, Seiten 199-204, XP001010139

## Beschreibung

Die vorliegende Erfindung betrifft die Verwendung einer Wirkstoffzusammensetzung in Haarpflegemitteln.

In der EP-A 227 994 sind bereits Verbindungen vom Ceramidtyp und deren Herstellung beschrieben, die als Feuchthaltemittel und Weichmacher in kosmetischen Mitteln wie Hautcremes, Lotionen, Lippenstiften, Hautreinigungsmitteln, Haarwässern, Haarkonditionern, Haarkuren und Haarwuchsmitteln eingesetzt werden sollen.

Deren Wirkungsmechanismus wird so gesehen, daß sie die Lipidmembran zwischen den Keratinzellen wiederherstellen und damit den Keratinschichten ein verbessertes Feuchtigkeitsrückhaltevermögen verleihen.

Ähnliche Ceramide und deren Verwendung in Haut- und Haarpflegemitteln sind Gegenstand der WO-A 96/37462.

Aus einem Artikel von K. De Paepe et al. im SÖFW-Jounal 122 (1996), S. 199-204, der auf einem Vortrag bei der IN-Cosmetics 1996 in Mailand zurückgeht, wird der Einfluß von Ceramiden, insbesondere Ceramide-3, im allgemeinen auf die Sperrfunktion und die Wasserretention der Haut untersucht, wobei auch die Möglichkeit der Verwendung von Gemischen aus Ceramiden, Fettsäuren und Cholesterin zur Verbesserung dieser Eigenschaften erörtert wird.

Daraus läßt sich jedoch nicht auf die Eignung zur Behandlung des anders strukturierten Haares schließen, bei dem diese Funktionen keine Rolle spielen.

Es wurde nunmehr gefunden, daß sich die Wirkung, Einarbeitbarkeit und Verträglichkeit dieser Ceramide in Haarpflegemitteln, wesentlich verbessern läßt, wenn man sie in Kombination mit mindestens einem Sterol, vorzugsweise einem Phytosterol, und mindestens einer C₁₂-C₂₄-Fettsäure einsetzt. Vorzugsweise enthält diese synergistische Mischung noch einen Polyalkohol wie 1,2-Propandiol und mindestens ein nichtionisches Tensid, insbesondere ein C₈-C₂₀-Alkylpolyglucosid.

Gegenstand der vorliegenden Erfindung ist also die Verwendung von Wirkstoff-Zusammensetzungen, enthaltend in wäßriger Grundlage eine Kombination aus
a) mindestens einem Ceramid der allgemeinen Formel I 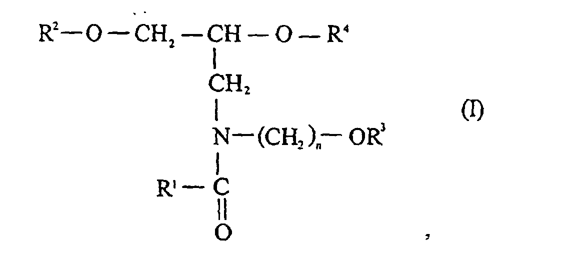 worin R¹ und R² gleiche oder verschiedene Alkyl- bzw. Alkenylreste mit 10 bis 22 Kohlenstoffatomen bedeuten, R³ für Wasserstoff oder eine Methyl-, Ethyl-, n-Propyloder Isopropylgruppe steht, R⁴ Wasserstoff, eine Hydroxymethyl-, Hydroxyethyl-, Dihydroxyethyl- oder Dihydroxypropylgruppe, und n eine ganze Zahl von 1 bis 6 bedeuten, vorzugsweise in einer Menge von 1 bis 25 Gew. -%;
b) mindestens einer C₁₀-C₂₄-Fettsäure, vorzugsweise in einer Menge von 1 bis 25 Gew.-%; und
c) mindestens einem Sterol, vorzugsweise in einer Menge von 1 bis 25 Gew.-%, in Haarpflegemitteln.

Vorzugsweise enthält die erfindungsgemäß verwendete Zusammensetzung noch etwa 1 bis 25 Gew.-% mindestens eines Polyalkohols (d), insbesondere 1,2-Propandiol.

Ein weiterer bevorzugter Bestandteil (e) in den erfindungsgemäß verwendeten Zusammensetzungen ist mindestens ein nichtionisches Tensid.
Als solches sind C₈-C₂₀-Alkylglucoside bevorzugt, vor allem solche mit einem Kondensationsgrad (z) von etwa 1,1 bis etwa 5, vorzugsweise in einer Menge von etwa 1 bis 25 Gew.-% der Zusammensetzung.

Das Gewichtsverhältnis der Komponenten a) zu b) liegt vorzugsweise bei etwa 1:4 bis 2:1, insbesondere 1:2,5 bis 1:1.

Das Gewichtsverhältnis der Komponenten a) zu c) beträgt vorzugsweise etwa 1:4 bis 4:1, insbesondere 1:2 bis 2:1, beispielsweise 1:1.
Das Gewichtsverhältnis der Komponenten b) zu c) schließlich liegt vorzugsweise bei 4:1 bis 1:2, insbesondere 2,5:1 bis 1:1.
Das Gewichtsverhältnis der Bestandteile a) und c) zu den Polyalkoholen d), soweit vorhanden, liegt vorzugsweise bei jeweils 5:1 bis 1:5, insbesondere 2:1 bis 1:3; gleiches gilt bezüglich des bevorzugten Gewichtsverhältnisses von jeweils a) und c) zum fakultativen Bestandteil nichtionisches Tensid e) und das Gewichtsverhältnis von diesem zum Polyalkohol d), sofern beide gemeinsam in der erfindungsgemäß verwendeten Zusammensetzungen anwesend sind.

Das Gewichtsverhältnis der C₁₂-C₂₄-Fettsäure, Bestandteil b), zu den fakultativen Bestandteilen d) und e) beträgt vorzugsweise jeweils 4:1 bis 1:3, insbesondere 2,5:1 bis 1:1.

Die Komponente a) des erfindungsgemäß eingesetzten Gemisches ist ein Ceramid der in Formel I definierten Struktur.
Die bevorzugten Gruppen R¹ und R² sind C₁₂-C₁₈-Alkylreste; n ist eine Zahl von 1 bis 3, R³ bedeutet vorzugsweise Wasserstoff oder einen Methylrest, und R⁴ Wasserstoff oder einen Dihydroxypropylrest.
Besonders bevorzugt sind Verbindungen, in denen R¹ einen C₁₂-C₂₄-Alkylrest, insbesondere eine C₁₃H₂₇-Alkylgruppe, R² einen C₁₄-C₁₈-Alkylrest, insbesondere eine C₁₆H₃₃-Alkylgruppe, R³ einen Methylrest, R⁴ eine 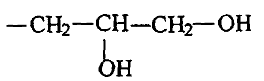 -Gruppe, und n 3 darstellen, oder eine Verbindung, wo R¹ für einen C₁₅-C₃₁-Alkylrest, R² für einen C₁₆-H₃₃-Alkylrest, R³ und R⁴ für je ein Wasserstoffatom und n für 2 stehen.

Der zweite essentielle Bestandteil b) der erfindungsgemäß eingesetzten Wirkstoffkombination ist mindestens eine C₁₀-C₂₄-Fettsäure, vorzugsweise eine C₁₂-C₂₂-Fettsäure.

Diese kann gesättigt oder ungesättigt, verzweigt oder unverzweigt sein; bevorzugt ist Behensäure.

Weitere geeignete Fettsäuren sind beispielsweise Laurinsäure, Myristinsäure, Ölsäure, Stearinsäure, Isostearinsäure oder auch Fettsäuregemische, beispielsweise natürliche wie Cocosfettsäuren und Talgfettsäuren oder synthetische, z.B. die über die Oxosynthese gewonnenen Fettsäuregemische.
Ihr Anteil im erfindungsgemäß verwendeten Wirkstoffgemisch liegt vorzugsweise bei etwa 1 bis 25, insbesondere etwa 2,5 bis 20, vor allem 5 bis 15 Gew.-% des Wirkstoffgemisches.

Der dritte essentielle Bestandteil der erfindungsgemäß verwendeten Kombination besteht aus mindestens einem Sterol.
Dieses kann tierischen Ursprungs sein (Zoosterine), z.B. Cholesterol und Lanosterol. Besonders bevorzugt sind jedoch Phytosterole, d.h., Sterole pflanzlichen Ursprungs. Als solche seinen beispielhaft Ergosterol, Sitosterol, Stigmasterol, Fucosterol, Brassicasterol, Fungisterol, Campesterol, Zymosterol, Ascosterol, Cerevisterol, Episterol, Faecosterol, Spinasterol oder auch Gemische von Phytosterolen, z.B. Sojasterol, genannt Ein im Rahmen der Erfindung besonders bevorzugtes Phytosterol ist "Avocadin", d.h., die in der unverseifbaren Fraktion des Avocadoöls vorliegenden Phytosterole.
Generell ist festzustellen, daß alle in pflanzlichen Fetten, Ölen und Wachsen vorhandenen Phytosterine bzw. Phytosteringemische zum erfindungsgemäßen Einsatz geeignet sind.

Deren Anteil im Wirkstoffgemisch liegt bevorzugt bei etwa 1 bis 25, insbesondere bei etwa 2,5 bis 20, insbesondere etwa 5 bis 15 Gew.-% der Wirkstoffkombination.

Der fakultative Bestandteil d) umfaßt mindestens einen Polyalkohol, wiederum in einer bevorzugten Menge von etwa 1 bis 25, insbesondere etwa 2,5 bis 20, vor allem etwa 5 bis 15 Gew.-%, der Wirkstoffkombination.
Bevorzugte Polyalkohole sind 1,2-Propandiol und Glycerin, jedoch sind auch weitere in kosmetischen Mitteln üblicherweise eingesetzte Polyalkohole wie beispielsweise 1,3-Butandiol, Sorbit oder Mannit geeignet.

Der weitere falkultative Bestandteil e), ein nichtionisches Tensid, ist in der erfindungsgemäß verwendeten Wirkstoffkombination vorzugsweise in einer Menge von etwa 1 bis 25, insbesondere etwa 2,5 bis 20, vor allem etwa 5 bis 15 Gew.-% enthalten.

Bevorzugte nichtionische Tenside sind insbesondere Alkylpolyglucoside der Formel

R-O-[G]_{z},

worin R eine C₈-C₂₀-Alkylgruppe, G einen Zuckerrest mit 5 bis 6 Kohlenstoffatomen, und z eine Zahl von 1,1 bis 5 bedeuten.
Es können auch weitere nichtionische Tenside, allein oder im Gemisch mit Alkylpolyglucosiden, eingesetzt werden, beispielsweise Fettalkoholethoxylate, Polyolfettsäureester, Sorbitanester und/oder Aminoxide.

Die erfindungsgemäß verwendete Wirkstoffmischung, die auch noch weitere in kosmetischen Mitteln übliche Wirk- und Zusatzstoffe enthalten kann, wird in Haarpflegemitteln eingesetzt.

Sie wirkt auf das Haar strukturverbessernd und ist insbesondere zur Behandlung und Regenerierung von strukturgeschädigtem Haar geeignet. Sie wirkt generell konditionierend auf das Haar und verbessert dessen Elastizität, die Naß- und Trockenkämmbarkeit und verleiht ihm erhöhten Glanz und Volumen.

Für den Einsatz der beschriebenen Kombination sind insbesondere Haarreinigungsmittel wie Shampoos, auch Tönungsshampoos, und vor allem Haarbehandlungsmittel wie Dauerwellmittel einschließlich Vor- und Nachbehandlungsmitteln, Haarfärbemittel auf Basis direktziehender und Oxidations-Farbstoffe, peroxidhaltige Fixiermittel für Dauerwellen bzw. Entwickler für Oxidationshaarfarben, Haarnachbehandlungsmittel, die entweder im Haar verbleiben oder nach der Behandlung ausgespült werden, beispielsweise Haarspülungen, die auch direktziehende Farbstoffe enthalten können, Haarkuren, Haarkonditioner, die zusätzlich die bekannten konditionierenden Wirkstoffe wie langkettige quaternäre Ammoniumververbindungen, anionische, kationische, amphotere und/oder nichtionische Polymere, Fette, Öle, etc. enthalten können, Haarwässer und Haarfestiger geeignet.

Diese beispielhaft genannten Mittel können auch als Aerosolpräparate, z.B. Aerosolschäume, vorliegen.

Die Zusammensetzung der Haarpflegemittel, in denen die erfindungsgemäße Kombination verwendet wird, ist grundsätzlich bekannt.
Es wird hierzu, zur Vermeidung von Wiederholungen, auf die umfassende Monographie von K. Schrader, Grundlagen und Rezepturen der Kosmetika, 2. Aufl. (1989, Hüthig Buchverlag), verwiesen.
Dort sind haarkosmetische Präparate, deren Zusammensetzung und Herstellung auf S. 677 bis 848 geoffenbart.

Die bei Schrader beispielhaft beschriebenen sowie generell die aus dem Stand der Technik bekannten Produkte sind grundsätzlich zur Aufnahme der erfindungsgemäß verwendeten Wirkstoffkombination geeignet.

Die Menge der Wirkstoffkombination in den Endprodukten hängt von deren Zusammensetzung und dem angestrebten Effekt ab.

Prinzipiell kommt ein Rahmen von etwa 0,01 bis etwa 25, vorzugsweise etwa 0,1 bis 20, insbesondere 0,5 bis 15 Gew.-%, bezogen auf das Endprodukt und den Wirkstoffgehalt der erfindungsgemäßen Zusammensetzung, in Betracht.

Der pH-Wert der Endprodukte liegt vorzugsweise im sauren bis leicht alkalischen Bereich bei etwa 2,5 bis etwa 8, vorzugsweise zwischen 3 und 7.

Die folgenden Beispiele dienen der Illustration der Erfindung.

Es wurden zwei erfindungsgemäße Wirkstoffkombinationen A-1 und A-2 der folgenden Zusammensetzung hergestellt:

### Zusammensetzung A-1:

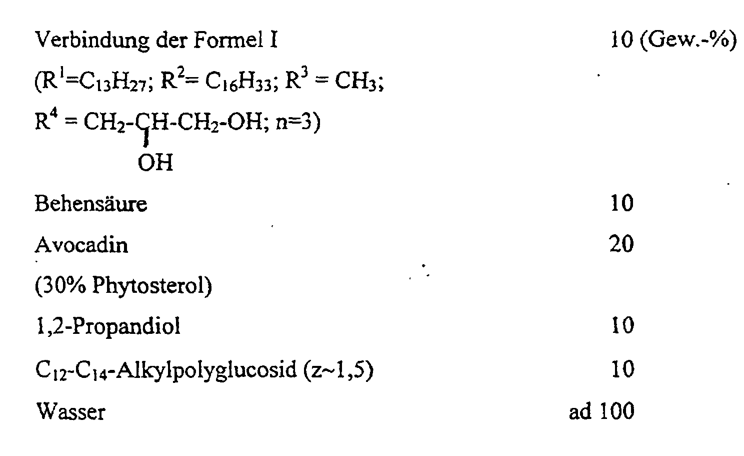

### Zusammensetzung A-2:

| | |
|---|---|
| Verbindung der Formel I (R¹=C₁₅H₃₁; R²=C₁₆H₃₃; R³=H; R⁴=H; n=2) | 10 (Gew.-%) |
| Behensäure | 10 |
| Avocadin | 10 |
| Sojasterin | 5 |
| 1,2-Propandiol | 10 |
| C₈-C₁₀-Alkylpolyglucosid (z ∼1,35) | 10 |
| Wasser | ad 100 |

### Beispiel 1

### Haarnachbehandlungsmittel

| | |
|---|---|
| Cetylstearylalkohol | 5,0 (Gew.-%) |
| Di-C₁₂-C₁₅-alkyldimethylammoniumchlorid | 2,0 |
| Stearyltrimethylammoniumchlorid | 2,0 |
| 1,2-Propandiol | 3,0 |
| Zusammensetzung A-1 | 0,5 |
| Benzyloxyethanol | 2,5 |
| Parfum, Konservierungsmittel | q.s. |
| Citronensäure / NaOH | ad pH 5 |
| Wasser | ad 100,0 |

Bei Anwendung dieser Zusammensetzung wurde ein weiches Haar mit angenehmem, vollen Griff, Sprungkraft und Elastizität erhalten.

### Beispiel 2

### Haarnachbehandlunsgmittel

| | |
|---|---|
| Cetylstearylalkohol | 5,0 (Gew.-%) |
| Di-C₁₂-C₁₅-alkyldimethylammoniumchlorid | 1,0 |
| Stearyltrimethylammoniumchlorid | 1,0 |
| Zusammensetzung A-2 | 5,0 |
| 1,2-Propandiol | 3,0 |
| Benzyloxyethanol | 2,5 |
| Parfum, Konservierungsmittel | q.s. |
| Citronensäure/NaOH | ad pH 5 |
| Wasser | ad 100,0 |

Diese Zusammensetzung ist besonders zur Behandlung und Regenerierung strukturgeschädigter Haare, geeignet.

### Beispiel 3

### Sprüh-Konditioniermittel für normales Haar

| | |
|---|---|
| Ethanol | 45,0 (Gew.-%) |
| Polyquaternium-6 | 0,1 |
| Zusammensetzung A-2 | 0,2 |
| PEG-160-hydriertes Ricinusöl | 0,5 |
| Parfum, Konservierungsmittel | q.s. |
| Citronensäure | ad pH 4,7 |
| Wasser | ad 100,0 |

Diese Zusammensetzung wird mit einer üblichen Pumpvorrichtung auf das Haar aufgebracht und bewirkt dort eine lockere, glänzende Kondition mit weichem, elastischem, vollem Griff.

### Beispiel 4

### Schaumkonditioniermittel

| | |
|---|---|
| Quaternium-80 | 0,2 (Gew.-%) |
| Polyquaternium-11 | 0,7 |
| Zusammensetzung A-1 | 0,2 |
| PEG-160-hydriertes Ricinusöl | 0,5 |
| Parfum, Konservierungsmittel | q.s. |
| Citronensäure | ad pH 5 |
| Wasser | ad 100,0 |

Diese Zusammensetzung wurde im Verhältnis 90:10 mit einem handelsüblichen Propan/Butan-Treibmittelgemisch abgefüllt. Das resultierende Schaumaerosol zeigte hervorragende haarkonditionierende Eigenschaften.

### Beispiel 5

### Haar- und Kopfhautlotion

| | |
|---|---|
| Acrylsäure /C₁₀-C₃₀-Alkylacrylat-Copolymer | 0,20 |
| Natriumhydroxid | 0,02 |
| Zusammensetzung A-2 | 0,20 |
| Panthenol | 0,05 |
| Isostearoyllactylat | 0,05 |
| Ethanol | 25,00 |
| PEG-160-hydriertes Ricinusöl | 0,30 |
| Parfum, Citronensäure | q.s. |
| Wasser | ad 100,0 |

Aufbringen dieses Haarwassers auf Haar und Kopfhaut verlieh dem Haar Glanz, Volumen und Elastizität.

### Beispiel 6

### Haargel

| | |
|---|---|
| Polyacrylsäure (Carbopol^{R}) | 1,00 (Gew.-%) |
| Natriumhydroxid | 0,03 |
| PPG-400 | 0,15 |
| Zusammensetzung A-1 | 0,15 |
| Vinylpyrrolidon/Vinylacetat-Copolymerisat (30:70) | 0,10 |
| Ethanol | 30,00 |
| Parfum | q.s. |
| Wasser | ad 100,0 |

Das Produkt wies exzellente haarfestigende und konditionierende Eigenschaften auf.

### Beispiel 7

### Konditionierendes Shampoo

| | |
|---|---|
| Natriumlaurylethersulfat | 10,0 (Gew.-%) |
| Decylglucosid | 2,0 |
| Polyquaternium- 10 | 0,5 |
| **oder** | |
| Polyquaternium-7 | 2,0 |
| Zusammensetzung A-2 | 0,2 |
| Natriumchlorid | 1,5 |
| Citronensäure | 0,2 |
| PEG-160-hydriertes Ricinusöl | 0,5 |
| Konservierungsmittel | 0,4 |
| Parfüm | 0,4 |
| Wasser | ad 100,0 |

Im Halbseitenversuch wurde an 10 Probanden jeweils eine Kopfhälfte mit einem Shampoo der obengenannten Zusammensetzung und die andere Kopfhälfte mit einer identischen Zusammensetzung, die jedoch die "Zusammensetzung A-2" nicht enthielt, gewaschen, die Naßkämmbarkeit beurteilt, und dann getrocknet.
Anschließend wurden die Trockenkämmbarkeit, der Glanz, das Volumen, die Elastizität und die Sprungkraft des Haares durch jeweils 2 Friseure beurteilt.
In allen beurteilten Kriterien ergab sich eine klare Überlegenheit des nach Beispiel 1 zusammengesetzten, die "Zusammensetzung A-2" enthaltenden Shampoos.

## Patentansprüche

1. Verwendung einer Wirkstoffzusammensetzung, enthaltend in wäßriger Grundlage eine Kombination aus
a) mindestens einem Ceramid der allgemeinen Formel I 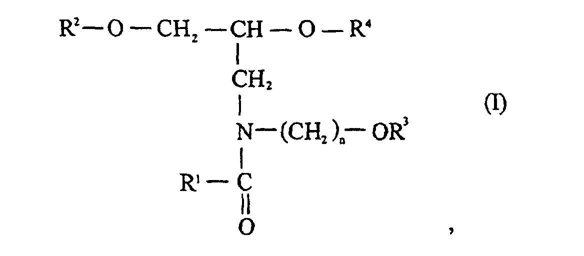 worin R¹ und R² gleiche oder verschiedene Alkyl- bzw. Alkenylreste mit 10 bis 22 Kohlenstoffatomen bedeuten, R³ für Wasserstoff oder eine Methyl-, Ethyl-, n-Propyloder Isopropylgruppe steht, R⁴ Wasserstoff, eine Hydroxymethyl-, Hydroxyethyl-, Dihydroxyethyl- oder Dihydroxypropylgruppe, und n eine ganze Zahl von 1 bis 6 bedeuten,
b) mindestens einer C₁₀-C₂₄-Fettsäure; und
c) mindestens einem Sterol
in Haarpflegemitteln.

2. Verwendung einer Zusammensetzung nach Anspruch 1, enthaltend in wäßriger Grundlage eine Kombination aus
a) 1 bis 25 Gew.-% mindestens eines Ceramids der allgemeinen Formel I;
b) 1 bis 25 Gew.-% mindestens einer C₁₀-C₂₄-Fettsäure;
c) 1 bis 25 Gew.-% mindestens eines Sterols,
jeweils berechnet auf die Gesamtzusammensetzung.

3. Verwendung einer Zusammensetzung nach Anspruch 1 oder 2, enthaltend 1 bis 25 Gew.-% mindestens eines Polyalkohols, berechnet auf die Gesamtzusammensetzung.

4. Verwendung einer Zusammensetzung nach Anspruch 3, enthaltend 2,5 bis 20 Gew.-% 1,2-Propandiol, berechnet auf die Gesamtzusammensetzung.

5. Verwendung einer Zusammensetzung nach einem oder mehreren der Ansprüche 1 bis 4, enthaltend 1 bis 25 Gew.-% mindestens eines nichtionischen Tensids, berechnet auf die Gesamtzusammensetzung.

6. Verwendung einer Zusammensetzung nach Anspruch 5, enthaltend 2,5 bis 15 Gew.-% mindestens eines C₈-C₂₀-Alkylpolyglucosids, berechnet auf die Gesamtzusammensetzung.

7. Verwendung einer Zusammensetzung nach einem oder mehreren der Ansprüche 1 bis 6, enthaltend 2,5 bis 15 Gew.-% mindestens eines Phytosterols, berechnet auf die Gesamtzusammensetzung.

8. Verwendung einer Zusammensetzung nach einem oder mehreren der Ansprüche 1 bis 7 **dadurch gekennzeichnet, daß** das Gewichtsverhältnis der Bestandteile a) zu b) etwa 1:4 bis 2:1 beträgt.

9. Verwendung einer Zusammensetzung nach einem oder mehreren der Ansprüche 1 bis 8, **dadurch gekennzeichnet, daß** das Gewichtsverhältnis der Bestandteile a) zu c) etwa 1:4 bis 4:1 beträgt.

10. Verwendung einer Zusammensetzung nach einem oder mehreren der Ansprüche 1 bis 9, **dadurch gekennzeichnet, daß** das Gewichtsverhältnis der Bestandteile b) zu c) etwa 4:1 bis 1:2 beträgt.

## Claims

1. Use of a Composition of active substances, comprising a combination of
a) at least one ceramide of the general formula I 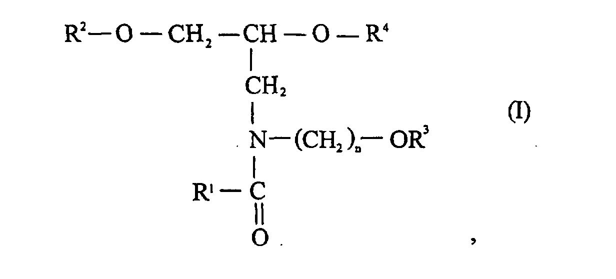 wherein R¹ and R² are identical or different alkyl or alkenyl groups with 10 to 22 carbon atoms, R³ is hydrogen or a methyl, ethyl, n-propyl or isopropyl group, R⁴ is hydrogen, a hydroxymethyl, hydroxyethyl, dihydroxyethyl, or dihydroxy propyl group and n is a number from 1 to 6,
b) at least one C₁₀-C₂₄-fatty acid; and
c) at least one sterol,
in hair treatment compositions.

2. Use of a composition according to claim 1, comprising a combination of
a) 1% to 25 % by weight of at least one ceramide of the general formula I;
b) 1% to 25 % by weight of at least one C₁₀-C₂₄-fatty acid;
c) 1% bis 25 % by weight of at least one sterol,
each calculated to the total composition.

3. Use of a composition according to claim 1 or 2, comprising 1% to 25 % by weight of at least one polyalcohol, calculated to the total composition.

4. Use of a composition according to claim 3, comprising 2.5% to 20% by weight of 1.2-propanediol, calculated to the total composition.

5. Use of a composition according to one or more of claims 1 to 4, comprising 1% to 25% by weight of at least one nonionic surfactant, calculated to the total composition.

6. Use of a composition according to claim 5, comprising 2.5% to 15% by weight of at least one C₈-C₂₀-alkyl polyglucoside, calculated to the total composition.

7. Composition according to one or more of claims 1 to 6, comprising 2.5% to 15% by weight of at least one phytosterol, calculated to the total composition.

8. Use of a composition according to one or more of claims 1 to 7, wherein the weight proportions of the components a) to b) amount to about 1:4 to 2:1.

9. Use of a composition according to one or more of claims 1 to 8, wherein the weight proportion of the components a) to c) amounts to about 1:4 to 4:1.

10. Use of a composition according to one or more of claims 1 to 9, wherein the weight proportion of the components b) to c) amounts to about 4:1 to 1:2.

## Revendications

1. Utilisation d'une composition de substance active qui contient dans une base aqueuse une combinaison de :
a) au moins un céramide de formule générale I : 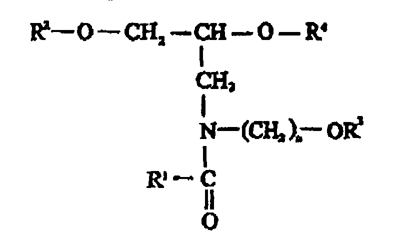 dans laquelle R¹ et R², identiques ou différents, représentent un résidu alkyle ou alcényle qui compte de 10 à 22 atomes de carbone, R³ représente l'hydrogène ou un groupe méthyle, éthyle, n-propyle ou isopropyle, R⁴ représente l'hydrogène, un groupe hydroxyméthyle, hydroxyéthyle, dihydroxyéthyle ou dihydroxypropyle et n représente un nombre entier compris entre 1 et 6,
b) au moins un acide gras en C₁₀ à C₂₄ et
c) au moins un stérol
dans des agents de soins des cheveux.

2. Utilisation d'une composition selon la revendication 1, qui contient dans une base aqueuse une combinaison constituée de :
a) de 1 à 25 % en poids d'au moins un céramide de formule générale I,
b) de 1 à 25 % en poids d'au moins un acide gras en C₁₀ à C₂₄,
c) de 1 à 25 % en poids d'au moins un stérol,
ces pourcentages étant chaque fois calculés par rapport à la composition totale.

3. Utilisation d'une composition selon la revendication 1 ou 2, qui contient de 1 à 25 % en poids d'au moins un alcool polyfonctionnel, ce pourcentage étant calculé par rapport à la composition totale.

4. Utilisation d'une composition selon la revendication 3, qui contient de 2,5 à 20 % en poids de 1,2-propane de diol, ce pourcentage étant calculé par rapport à la composition totale.

5. Utilisation d'une composition selon l'une ou plusieurs des revendications 1 à 4, qui contient de 1 à 25 % en poids d'au moins un agent tensioactif non ionique, ce pourcentage étant calculé par rapport à la composition totale.

6. Utilisation d'une composition selon la revendication 5, qui contient de 2,5 à 15 % en poids d'au moins un (alkyle en C₈ à C₂₀)polyglucoside, ce pourcentage étant calculé par rapport à la composition totale.

7. Utilisation d'une composition selon l'une ou plusieurs des revendications 1 à 6, qui contient de 2,5 à 15 % en poids d'au moins un phytostérol, ce pourcentage étant calculé par rapport à la composition totale.

8. Utilisation d'une composition selon l'une ou plusieurs des revendications 1 à 7, **caractérisée en ce que** le rapport pondéral entre les composants a) et b) est compris entre environ 1:4 et 2:1.

9. Utilisation d'une composition selon l'une ou plusieurs des revendications 1 à 8, **caractérisée en ce que** le rapport pondéral entre les composants a) et c) est compris sensiblement entre 1:4 et 4:1.

10. Utilisation d'une composition selon l'une ou plusieurs des revendications 1 à 9, **caractérisée en ce que** le rapport pondéral entre les composants b) et c) est compris entre environ 4:1 et 1:2.
